# EUROPÄISCHE PATENTANMELDUNG

(11) **EP 0 821 975 A1**
(43) Veröffentlichungstag der Anmeldung: **04.02.1998**
(21) Anmeldenummer: 96810512.2
(22) Anmeldetag: 31.07.1996
(51) Int. Cl.: A61M 11/00, B05B 11/00

(54) **Dosiervorrichtung für fliessfähige Stoffe**

(71) Anmelder: Novartis AG (Novartis SA) (Novartis Inc.), 4058 Basel (CH)
(72) Erfinder: Die Erfindernennung liegt noch nicht vor

(57) **Zusammenfassung**

Die Dosiervorrichtung (1, 31) umfass einen Vorratsbehälter (16, 36) für den zu dosierenden Stoff und eine den Stoff zur Abgabeöffnung (3) der Dosiervorrichtung transportierende Fördereinrichtung (5). Der Vorratsbehälter (16, 36) ist allseitig nach aussen abgedichtet mit der Fördereinrichtung (5) verbunden und in der Wandung des Vorratsbehälters (16, 36) ist ein Verschlussstück (21, 41) vorgesehen, auf das beim Entleeren des Vorratsbehälters (16, 36) infolge des äusseren Luftdruckes eine Kraft einwirkt, durch die die Form des Vorratsbehälters (16, 36) im Bereich des Verschlussstückes (21, 41) veränderbar ist. Ferner ist eine der Bewegung des Verschlussstückes (21, 41) zugeordnete Anzeigeeinrichtung (18, 38) vorgesehen, durch die das Ausmass der Bewegung des Verschlussstückes (21, 41) und damit die abgegebene Stoffmenge anzeigbar ist.

## Beschreibung

Die Erfindung betrifft eine Dosiervorrichtung für fliessfähige Stoffe, insbesondere zu versprühende Flüssigkeiten, mit einem Vorratsbehälter für den zu dosierenden Stoff und einer den Stoff zur Abgabeöffnung der Dosiervorrichtung transportierenden Fördereinrichtung.

Derartige Dosiervorrichtungen sind in Gestalt von Pumpspraysystemen bekannt, die es gestatten, Flüssigkeiten wie z.B. Arzneistofflösungen zu versprühen. Die bekannten Systeme müssen zuerst durch Füllen der Dosierpumpe aktiviert werden, wobei vom Benutzer nicht kontrolliert werden kann, ab welchem Zeitpunkt die Dosierpumpe vollständig gefüllt ist und daher ab welchem Zeitpunkt die volle einer Pumpbewegung zugeordnete Lösungsmenge versprüht wird. Es ist daher mit den bekannten Pumpspraysystemen nicht möglich, die von den Registrierungsbehörden für Arzneimittel geforderte Dosiergenauigkeit von fünf Prozent mit den auf dem Markt befindlichen Dosierpumpen zu erreichen.

Ferner gestatten die bisher bekannten Pumpspraysysteme nicht, eine Trockensubstanz erst kurz vor Anwendung am Patienten in einem geeigneten Lösungsmittel aufzulösen und danach aus einem gasfreien Reservoir heraus exakt und steril zu applizieren.

Der Erfindung liegt die Aufgabe zugrunde, eine Dosiervorrichtung zu schaffen, die auf einfache Weise leicht anwendbar ist und auch an schwierig zugänglichen Stellen eine quantitativ exakte und sterile Applikation einer Wirkstofflösung gestattet.

Diese Aufgabe wird erfindungsgemäss bei einer Dosiervorrichtung der eingangs genannten Art dadurch gelöst, dass der Vorratsbehälter allseitig nach aussen abgedichtet mit der Fördereinrichtung verbunden ist und in der Wandung des Vorratsbehälters ein Verschlussstück vorgesehen ist, auf das beim Entleeren des Vorratsbehälters infolge des äusseren Luftdruckes eine Kraft einwirkt, durch die die Form des Vorratsbehälters im Bereich des Verschlussstückes veränderbar ist und dass eine der Bewegung des Verschlussstückes zugeordnete Anzeigeeinrichtung vorgesehen ist, durch die das Ausmass der Bewegung des Verschlussstückes und damit die abgegebene Stoffmenge anzeigbar ist.

Als Fördereinrichtung ist besonders zweckmässig, eine Dosierpumpe für ein gasfrei verschlossenes System zu verwenden, die über eine Stossbewegung eines zugeordneten Sprühkopfes betätigbar ist. Bei einem zweckmässigen Ausführungsbeispiel ist der Vorratsbehälter durch den Spritzenkörper einer Kolbenspritze und das Verschlussstück durch den Spritzenkolben der Kolbenspritze gebildet.

Durch die dem als Verschlussstück dienenden Spritzenkolben zugeordnete Anzeigevorrichtung wird es ermöglicht, unabhängig von der Dosiergenauigkeit der Dosierpumpe die von den Registrierungsbehörden für Arzneimittel geforderte Dosiergenauigkeit von fünf Prozent zu erreichen, da die Dosiermenge nicht mehr durch Zählen der Stösse der Dosierpumpe erfasst wird sondern durch eine Ablesung an der dem Verschlussstück, insbesondere dem Spritzenkolben zugeordneten Skalierung.

Bei einem zweckmässigen Ausführungsbeispiel der Erfindung ist die Kolbenstange der Kolbenspritze lösbar mit dem Spritzenkolben verbunden, um Behinderungen durch die Kolbenstange zu vermeiden.

Wenn der Vorratsbehälter nicht insgesamt aus einem transparenten Material hergestellt ist, ist es zweckmässig, wenn die jeweilige Lage des Kolbens durch einen transparenten Bereich im zylindrischen Abschnitt des Vorratsbehälters von aussen erkennbar ist und der transparente Bereich mit der Skalierung versehen ist. Im einfachsten Fall besteht der Vorratsbehälter aus einer mit einer aufgedruckten Skalierung versehenen transparenten Fertigspritze.

Es ist jedoch auch möglich, den Vorratsbehälter als erste Kammer einer Doppelkammerspritze auszubilden, die den zu rekonstituierenden Wirkstoff enthält und wobei die Rekonstitutionsflüssigkeit aus der zweiten Kammer der Doppelkammerspritze vor Gebrauch in die erste Kammer injiziert wird.

Bei einer Dosiervorrichtung mit einer Einkammerspritze ist es zweckmässig, die Kolbenspritze lösbar mit der Dosierpumpe zu verbinden, indem ein Luer-Lock-Konus am vorderen Ende der Fertigspritze vorgesehen ist. Dies ermöglicht es, die Arzneistofflösung erst kurz vor der Anwendung frisch herzustellen, z.B. durch Auflösen einer Trockensubstanz mit einem geeigneten Lösungsmittel.

Obwohl die Erfindung nachfolgend anhand eines Ausführungsbeispiels für eine Dosiervorrichtung für zu versprühende Flüssigkeiten beschrieben wird, kann diese auch auf andere fliessfähige Stoffe, insbesondere Pasten, Gels und Cremes angewendet werden.

Nachfolgend werden Ausführungsbeispiele der Erfindung anhand der Zeichnung erläutert. Es zeigen:
- Fig. 1: ein Ausführungsbeispiel für eine Dosiervorrichtung gemäss der Erfindung vor Beginn der Applikation des Wirkstoffes in einem Längsschnitt,
- Fig. 2: die Dosiervorrichtung gemäss Fig. 1 nach der Applikation des Wirkstoffes und
- Fig. 3: eine Vorrichtung gemäss der Erfindung mit einem Aufbau entsprechend einer Doppelkammerspritze im Längsschnitt.

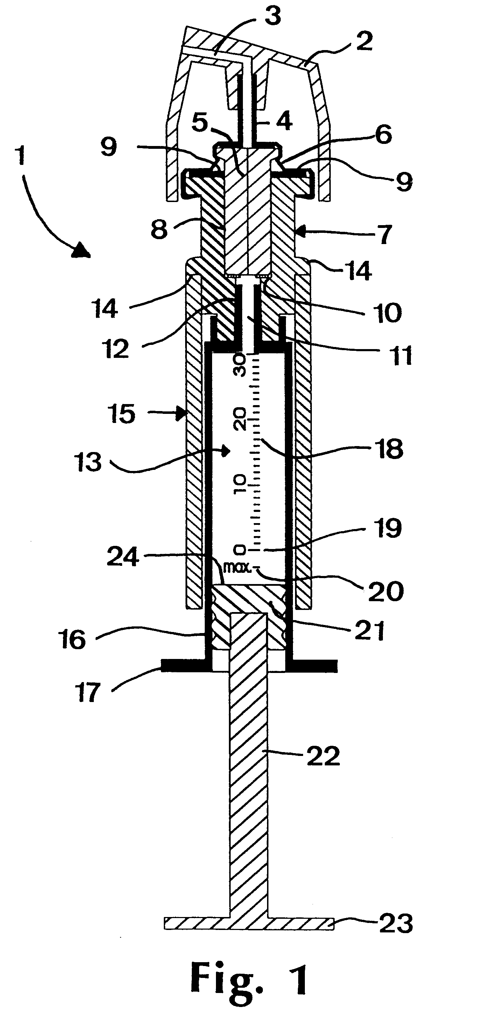

Die in Fig. 1 dargestellte Dosiervorrichtung 1 für fliessfähige Stoffe ist vorgesehen, um eine wundflächengerechte, topische und sterile Applikation von TGF-beta 3 (Transforming Growth Factor beta 3) durchzuführen. Mit Hilfe der in Fig. 1 dargestellten Dosiervorrichtung 1 soll der Wirkstoff in eine für die lokale topische Behandlung chronischer Wunden wie z.B. Ulcus cruris, Decubitus-Ulcera und Diabetes-Ulcera geeignete Darreichungsform gebracht werden, um ein patientenfreundliches System bereitzustellen.

Die Dosiervorrichtung 1 verfügt über einen Sprühkopf 2, der durch mechanische Zerteilung eines Flüssigkeitsstroms einen trägergasfreien Spray abgibt. Für obengenannten Anwendungszweck ist ein sogenannter "feuchter Spray" mit Teilchengrössen über 50 µm ideal. Der Sprühkopf 2 verfügt über einen Sprühkanal 3, der auf die Viskosität der verwendeten Formulierung des zu zerstäubenden Stoffes abgestimmt ist.

Der Sprühkanal 3 ist mit einem Pumpenröhrchen 4 einer Dosierpumpe 5 verbunden. Bei der Dosierpumpe 5 handelt es sich um eine Dosierpumpe für ein gasfrei verschlossenes System.

Durch stossweises Betätigen des Sprühkopfes 2 in axialer Richtung des Pumpenröhrchens 4 wird die Dosierpumpe 5 betätigt und dadurch über das Pumpenröhrchen 4 und den Sprühkanal 3 die gepumpte Flüssigkeit zerstäubt.

Die Dosierpumpe 5 ist z.B. mit Hilfe einer Metallkappe 6 mit einem Verbindungsstück 7 verbunden. Das Verbindungsstück 7 verfügt über eine zylindrische Bohrung 8, in der die zylindrische Dosierpumpe 5 mit Hilfe der aufgebördelten Metallkappe 6 gut dichtend verbunden ist. Zwischen der Metallkappe 6 und der Stirnseite des Verbindungsstücks 7 kann ein Dichtring 9 vorgesehen sein. Eine weitere Dichtung 10, beispielsweise aus Gummi, ist zur Abdichtung der Dosierpumpe 5 an einem Absatz 10 vorgesehen, gegen den die Dosierpumpe 5 abgestützt ist und an dem die zylindrische Bohrung 8 von einem der Dosierpumpe 5 angepassten grösseren Durchmesser in einen Bohrungsabschnitt 11 geringeren Durchmessers übergeht.

Der Bohrungsabschnitt 11 des im wesentlichen zylindrisch gestalteten Verbindungsstücks 7 dient zur abgedichteten Aufnahme des Kanülenanschlusses 12 einer Kolbenspritze 13.

Vorzugsweise ist die Verbindung zwischen der Kolbenspritze 13 und dem Verbindungsstück 7 durch eine in der Zeichnung schematisch dargestellte Kopplung mit Hilfe eines Luer-Lock-Konus realisiert. Auf diese Weise wird eine sichere und dichte, aber dennoch leicht lösbare Verbindung zwischen der Kolbenspritze 13 und dem Verbindungsstück 7 erreicht.

Das Verbindungsstück 7 verfügt über einen umlaufenden Anschlagring 14, gegen den ein transparentes Rohrstück 15 anschlägt, das fest mit dem Verbindungsstück 7 verbunden ist und das zur Aufnahme und Zentrierung der Kolbenspritze 13 dient, um eine leichtere Handhabung der Dosiervorrichtung 1 zu ermöglichen. In axialer Richtung erstreckt sich das Rohrstück 15 über eine Länge, die bis in die Nähe der am Spritzenkörper 16 angeformten Flanschteile 17 ragt.

Der Spritzenkörper 16 der Kolbenspritze 13 ist vorzugsweise ebenfalls aus einem transparenten Material hergestellt. Die Kolbenspritze 13 kann eine vorgefüllte Fertigspritze sein und enthält zu dem in Fig. 1 dargestellten Zeitpunkt die jeweils erforderliche spezifische pharmazeutische Wirkstofflösung.

Bei einem in der Zeichnung nicht dargestellten Ausführungsbeispiel enthält die Kolbenspritze 13 statt einer Flüssigkeit ein Gel oder eine Creme, wobei der Sprühkopf 2 und der Sprühkanal in diesem Falls so abgewandelt sind, dass keine Zerstäubung sondern lediglich ein präzises Austragen erfolgt.

Wie man in der Fig. 1 erkennt, ist durch das transparente Rohrstück 15 hindurch eine auf den Spritzenkörper 16 aufgedruckte Skalierung 18 sichtbar. Die Skalierung 18 dient zur Anzeige des bei der Betätigung der Dosierpumpe 5 dem Innenraum des Spritzenkörpers 16 entnommenen Volumens und damit der Dosis der spezifischen Wirkstofflösung. Die Skalierung 18 kann dabei den zu behandelnden Flächeneinheiten auf der Haut des Patienten zugeordnet sein.

Unterhalb der Nullmarke 19 der Skalierung 18 ist in Fig. 1 eine mit max. gekennzeichnete Markierung 20 zu erkennen, die dem optimalen maximalen Füllzustand der Kolbenspritze 13 beim Verbinden der Kolbenspritze 13 mit dem Verbindungsstück 7 und der Dosierpumpe 5 entspricht. Zur besseren Lesbarkeit ist in Fig. 1 der Spritzenkolben 21 der Kolbenspritze 13 in einer etwas weiter zurückliegenden Position als der optimalen Position beim Verbinden des Kanülenanschlusses 12 mit dem Bohrungsabschnitt 11 dargestellt.

Der Spritzenkolben 21 ist lösbar mit einer Kolbenstange 22, beispielsweise einer abschraubbaren Kolbenstange 22, verbunden. Die Kolbenstange 22 verfügt über ein Griffteil 23, mit dessen Hilfe der Spritzenkolben 21 manuell betätigbar ist.

Nach dem Einsetzen der bis zu der Markierung 20 gefüllten Kolbenspritze 13 in das Rohrstück 15 und dem dichten Verbinden des Kanülenanschlusses 12 mit der Eingangsseite der Dosierpumpe 5 kann die Kolbenstange 22 bei dem bevorzugten Ausführungsbeispiel der Erfindung aus dem Spritzenkolben 21 herausgeschraubt werden, so dass auf den Spritzenkolben 21 von aussen nicht mehr versehentlich eingewirkt werden kann.

Bevor die in Fig. 1 dargestellte Dosiervorrichtung 1 beim Patienten angewendet wird, erfolgt eine Kalibrierung der Dosiervorrichtung 1 durch mehrfaches Betätigen des Sprühkopfes 2 in axialer Richtung. Hierbei wird die Dosierpumpe 5 aktiviert. Sie ist nach wenigen Pumpenstössen ebenso wie der Innenraum des Spritzenkörpers 16 vollständig mit Flüssigkeit gefüllt und von Gasresten, insbesondere Luftresten, völlig befreit. Auf diese Weise lässt sich beim erstmaligen Benutzen oder auch nach längeren Standzeiten der Dosiervorrichtung 1 das Auftreten von Fehlmengen während des Sprühvorgangs vermeiden.

Hierzu ist es bei erstmaliger Benutzung der Dosiervorrichtung 1 für einen bestimmten Spritzeninhalt lediglich erforderlich, das Betätigen der Dosierpumpe 5 vor dem Applikationsvorgang dann zu beenden, wenn die in Fig. 1 nach oben weisende Stempelfläche 24 des Spritzenkolbens 21 mit der Nullmarke 19 fluchtet. Sobald dies der Fall ist, kann die Dosiervorrichtung 1 eingesetzt werden, um eine quantitativ exakte sterile Applikation der Wirkstofflösung durchzuführen.

Dabei ist es ohne Bedeutung, ob einzelne Pumpstösse zu unterschiedlichen Abgabemengen führen, da die über den Sprühkopf 2 abgegebene Menge der Wirkstofflösung durch die Volumenverringerung des Inhalts des Spritzenkörpers 16 direkt messbar ist. Die durch die Abgabe der Wirkstofflösung bewirkte Verringerung der Flüssigkeitsmenge im Spritzenkörper 16 führt aufgrund des äusseren Luftdruckes nämlich zu einer axialen Verschiebung des Spritzenkolbens 21 in Richtung auf deren Kanülenanschluss 12.

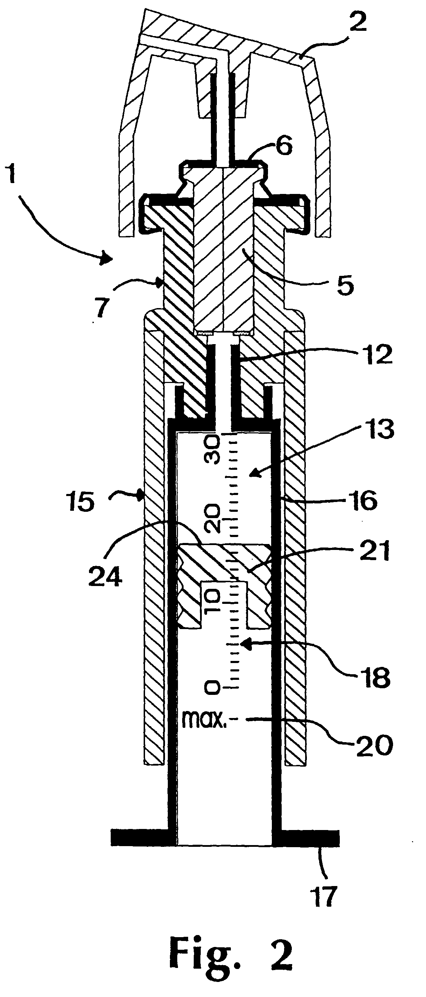

Die Darstellung in Fig. 2 zeigt die Dosiervorrichtung 1 nach dem Ende der Pumpbewegungen. Der gut gleitend und dichtend angeordnete Spritzenkolben 21 befindet sich mit seiner Stempelfläche 24 an einer Stelle der Skalierung 18, durch die genau angezeigt wird, welche Menge der Wirkstofflösung appliziert worden ist. Falls diese Menge noch nicht dem Sollwert entspricht, kann durch weitere Pumpenstösse, insbesondere auch Pumpenstösse, deren Hub nicht einem vollen Hub entspricht, genau die für erforderlich gehaltene Menge Spray erzeugt werden.

Fig. 2 zeigt den Spritzenkolben 21 am Ende einer Behandlung, bei der beispielsweise eine Fläche von 17 Flächeneinheiten behandelt werden sollte oder bei der eine Menge von 17 Volumeneinheiten aufgesprüht werden sollte.

Die pharmazeutische Wirkstofflösung kann in Form einer sterilen, vorgefüllten Fertigspritze bereitgestellt werden, die vor dem Gebrauch in die Dosiervorrichtung 1 eingesetzt wird. Soll die Lösung eines gefriergetrockneten Präparates versprüht werden, kann das Füllen der Kolbenspritze 13 vor deren Einsetzen in die Dosiervorrichtung 1 in der Weise durchgeführt werden, dass als Kolbenspritze 13 eine vorgefüllte Fertigspritze verwendet wird, auf deren Kanülenanschluss 12 zunächst eine Kanüle aufgesteckt wird. Mit Hilfe der Kanüle wird der Verschluss eines Injektionsfläschchens mit einem Lyophilisat durchstossen. Das Lyophilisat enthält die zur Applikation vorgesehenen Wirkstoffe in einer stabilen und biologisch aktiven Form. Nach dem Injizieren des Spritzeninhalts in das Injektionsfläschchen wird das Lyophilisat durch leichtes Schütteln beziehungsweise Umschwenken rekonstituiert. Anschliessend wird der Inhalt des Injektionsfläschchens wieder in die Kolbenspritze 13 aufgezogen. Zuviel aufgezogenen Lösung wird wieder ausgedrückt, bis sich die Stempelfläche 24 des Spritzenkolbens 21 unter der auf dem Spritzenkörper 16 angebrachten Markierung 20 befindet.

Danach wird die Kanüle abgezogen und die Kolbenspritze 13 in das Rohrstück 15 der Dosiervorrichtung 1 eingeführt und der Kanülenanschluss 12 dichtend mit dem Verbindungsstück 7 verbunden. Zur besseren Handhabung wird dabei in der aus der Fig. 2 ersichtlichen Weise die Kolbenstange 22 vom Spritzenkolben 21 abgeschraubt. In der oben erläuterten Weise kann durch mehrmaliges Drücken auf den Sprühkopf 2 die Dosierpumpe 5 gefüllt werden und dadurch der Spritzenkolben 21 langsam in Richtung auf den Kanülenanschluss 12 bewegt werden. Das Drücken wird so lange wiederholt, bis der Rand der Stempelfläche 24 auf der Nullmarke 19 der Skalierung 18 zu liegen kommt.

Die Dosiervorrichtung 1 ist nun gebrauchsfertig, und die Behandlung des Patienten kann beginnen, wobei zunächst die Grösse der zu behandelnden Fläche bestimmt wird. Die zu behandelnden Flächen des Patienten werden eingesprüht und die entnommene Dosis an der Skalierung 18 abgelesen. Nach dem Ende der Behandlung kann die gebrauchte Dosiervorrichtung 1 entsorgt werden.

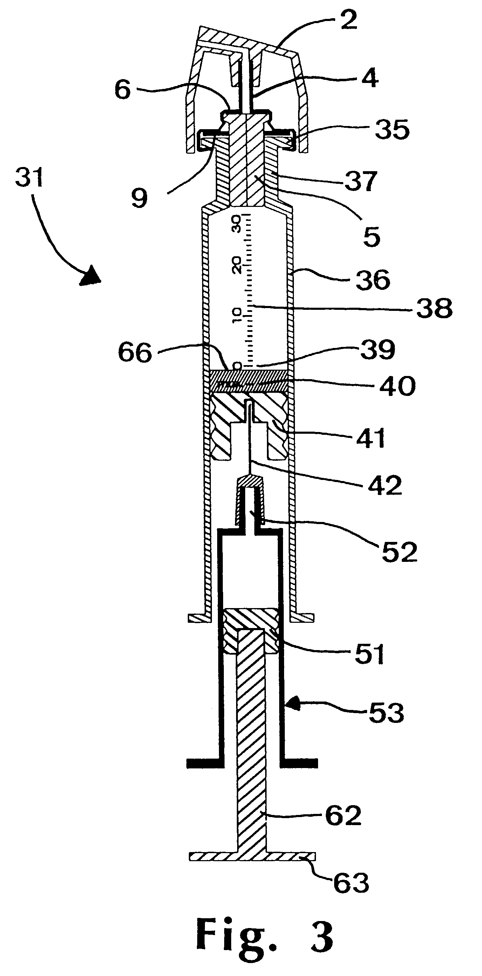

Bei dem in Fig. 3 dargestellten Ausführungsbeispiel der Erfindung ist die Dosiervorrichtung 31 in der Art einer Doppelkammerspritze aufgebaut. Die Dosierpumpe 5 ist in der aus den Fig. 1 und 2 bekannten Weise über ein Pumpenröhrchen 4 mit einem Sprühkopf 2 verbunden.

Die Dosierpumpe 5 ist mit Hilfe der Metallkappe 6 an einem Verbindungsstück 37 befestigt, das am vorderen Ende eines Spritzenkörpers 36 ausgebildet ist. Die Metallkappe 6 umbördelt ein Flanschteil 35 am vorderen Ende des Verbindungsstücks 37. Ein Dichtring 9 sorgt für eine gute Abdichtung.

Auf dem Spritzenkörper 36 ist eine Skalierung 38 vorgesehen, die in ihrer Funktion mit der Skalierung 18 bei dem in Fig. 1 und 2 dargestellten Ausführungsbeispiel übereinstimmt. In entsprechender Weise sind eine Nullmarke 39 und eine Markierung 40 für die maximale Füllung vorgesehen.

Im Spritzenkörper 36 ist ein Spritzenkolben 41 angeordnet, der mit Hilfe einer Kanüle 42 durchstossen werden kann. Die Kanüle 42 ist mit dem Kanülenanschluss 52 einer Kolbenspritze 53 verbunden.

Die Abmessungen der oben erwähnten Teile ist so gewählt, dass die Länge des Verbindungsstückes 37 wesentlich grösser als die Länge der Skalierung 38 ist und daher die Kolbenspritze 53 etwa über die Hälfte der Länge des Verbindungsstücks 37 und etwa über die Hälfte ihrer eigenen Länge in das Verbindungsstück 37 hineinragt, wenn sich der Spritzenkolben 41 in der Nähe der Markierung 40 befindet.

Die Kolbenspritze 53 verfügt über einen Spritzenkolben 51 und eine mit diesem verbundene Kolbenstange 62 mit Griffteil 63.

Mit Hilfe der als vorgefüllte Fertigspritze ausgebildeten Kolbenspritze 53 wird zu Beginn des Einsatzes der Dosiervorrichtung 31 über die Kanüle 42 eine Rekonstitutionsflüssigkeit durch den Spritzenkolben 41 hindurch in den Aufnahmeraum des Spritzenkörpers 36 injiziert. In Fig. 3 erkennt man ein Lyophilisat 66, welches durch leichtes Schütteln beziehungsweise Umschwenken aufgelöst wird. Beim Injizieren der Rekonstitutionsflüssigkeit wandert der Spritzenkolben 41 ausgehend von einer in Fig. 3 oberen Lage nach unten bis in eine Lage, bei der sich der Spritzenkolben 41 in der Nähe der Markierung 40 befindet. Wenn der Füllvorgang beendet ist, kann die Kolbenspritze 53 entfernt werden und die Dosiervorrichtung 31 entsprechend der Dosiervorrichtung 1 für die Applikation eines Wirkstoffes auf die Haut eines Patienten verwendet werden.

## Patentansprüche

1. Dosiervorrichtung (1, 31) für fliessfähige Stoffe, insbesondere zu versprühende Flüssigkeiten, mit einem Vorratsbehälter (16, 36) für den zu dosierenden Stoff und einer den Stoff zur Abgabeöffnung (3) der Dosiervorrichtung transportierenden Fördereinrichtung (5), **dadurch gekennzeichnet**, dass der Vorratsbehälter (16, 36) allseitig nach aussen abgedichtet mit der Fördereinrichtung (5) verbunden ist und in der Wandung des Vorratsbehälters (16, 36) ein Verschlussstück (21, 41) vorgesehen ist, auf das beim Entleeren des Vorratsbehälters (16, 36) infolge des äusseren Luftdruckes eine Kraft einwirkt, durch die die Form des Vorratsbehälters (16, 36) im Bereich des Verschlussstückes (21, 41) veränderbar ist und dass eine der Bewegung des Verschlussstückes (21, 41) zugeordnete Anzeigeeinrichtung (18, 38) vorgesehen ist, durch die das Ausmass der Bewegung des Verschlussstückes (21, 41) und damit die abgegebene Stoffmenge anzeigbar ist.

2. Vorrichtung nach Anspruch 1, dadurch gekennzeichnet, dass der Vorratsbehälter einen zylindrischen Abschnitt (16, 36) aufweist, in dem ein als Verschlussstück entlang der Längsachse des zylindrischen Abschnitts (16, 36) verschiebbarer Kolben (21, 41) angeordnet ist.

3. Vorrichtung nach Anspruch 2, dadurch gekennzeichnet, dass der Vorratsbehälter durch den Spritzenkörper (16, 36) einer Kolbenspritze (13, 53) und das Verschlussstück durch den Spritzenkolben (21, 41) der Kolbenspritze (13, 53) gebildet ist.

4. Vorrichtung nach Anspruch 3, dadurch gekennzeichnet, dass die Kolbenstange (22) der Kolbenspritze (13) lösbar mit dem Spritzenkolben (21) verbunden ist.

5. Vorrichtung nach einem der Ansprüche 2 bis 4, dadurch gekennzeichnet, dass die jeweilige Lage des Kolbens (21, 41) durch einen transparenten Bereich im zylindrischen Abschnitt (16, 36) des Vorratsbehälters von aussen erkennbar ist und dem transparenten Bereich (16, 36) eine Skalierung (18, 38) zugeordnet ist.

6. Vorrichtung nach einem der Ansprüche 1 bis 5, dadurch gekennzeichnet, dass der Vorratsbehälter eine mit einer aufgedruckten Skalierung versehene transparente Fertigspritze (13) ist.

7. Vorrichtung nach einem der Ansprüche 1 bis 5, dadurch gekennzeichnet, dass der Vorratsbehälter durch die erste Kammer (36) einer Doppelkammerspritze (36, 51) gebildet ist.

8. Vorrichtung nach einem der Ansprüche 3 bis 6, dadurch gekennzeichnet, dass die Kolbenspritze (13) lösbar mit der Fördereinrichtung (5) verbindbar ist.

9. Vorrichtung nach Anspruch 8, dadurch gekennzeichnet, dass die Kolbenspritze (13) mit Hilfe eines Luer-Lock-Konus mit der Fördereinrichtung (5) verbunden ist.

10. Vorrichtung nach einem der Ansprüche 1 bis 9, dadurch gekennzeichnet, dass die Fördereinrichtung eine Dosierpumpe (5) für ein gasfrei verschlossenes System ist, die über die Stossbewegung eines zugeordneten Sprühkopfes (2) betätigbar ist.
